# EUROPEAN PATENT SPECIFICATION

(11) **EP 4 367 513 B1**
(45) Date of publication and mention of the grant of the patent: **22.04.2026**
(21) Application number: 22743921.3
(22) Date of filing: 05.07.2022
(51) Int. Cl.: G01N 30/86, G01N 33/68, H01J 49/00, G01N 30/72, G01N 30/88

(54) **SCOUT MRM FOR SCREENING AND DIAGNOSTIC ASSAYS**
SCOUT MRM FÜR EINE VORRICHTUNG UND EIN VERFAHREN FÜR DIAGNOSTISCHEN UND WIRKSTOFF-SCREENING
SCOUT MRM POUR MÉTHODE ET SYSTÈME DE CRIBLAGE ET DE DIAGNOSTIQUE

(30) Priority: 08.07.2021 US 202163219425 P
(43) Date of publication of application: 15.05.2024
(73) Proprietor: DH Technologies Development Pte. Ltd., Singapore 739256 (SG)
(72) Inventor: PURKAYASTHA, Subhasish, Framingham, Massachusetts 01701 (US); LE BLANC, Yves, Concord, Ontario L4K 4V8 (CA)
(74) Representative: Maiwald GmbH
(86) International application number: PCT/IB2022/056193
(87) International publication number: WO 2023/281387

(56) References cited:
- WO-A1-2017/093861
- BLANDINE ROUGEMONT ET AL: "Scout-MRM: Multiplexed Targeted Mass Spectrometry-Based Assay without Retention Time Scheduling Exemplified by Dickeya dadantii Proteomic Analysis during Plant Infection", ANALYTICAL CHEMISTRY, vol. 89, no. 3, 9 January 2017 (2017-01-09), US, pages 1421 - 1426, XP055596027, ISSN: 0003-2700, DOI: 10.1021/acs.analchem.6b03201
- LEPRÊTRE MAXIME ET AL: "From shotgun to targeted proteomics: rapid Scout-MRM assay development for monitoring potential immunomarkers in", ANALYTICAL AND BIOANALYTICAL CHEMISTRY, SPRINGER BERLIN HEIDELBERG, BERLIN/HEIDELBERG, vol. 412, no. 26, 17 August 2020 (2020-08-17), pages 7333 - 7347, XP037245932, ISSN: 1618-2642, [retrieved on 20200817], DOI: 10.1007/S00216-020-02868-2
- FAUGERE JULIEN ET AL: "High-multiplexed monitoring of protein biomarkers in the sentinel Gammarus fossarum by targeted scout-MRM assay, a new vision for ecotoxicoproteomics", JOURNAL OF PROTEOMICS, ELSEVIER, AMSTERDAM, NL, vol. 226, 12 July 2020 (2020-07-12), XP086242750, ISSN: 1874-3919, [retrieved on 20200712], DOI: 10.1016/J.JPROT.2020.103901
- ANDREW JAMES ET AL: "Basic Design of MRM Assays for Peptide Quantification", 1 January 2010, LC-MS/MS IN PROTEOMICS PART III - METHODS AND APPLICATIONS; [METHODS IN MOLECULAR BIOLOGY, ISSN 1064-3745, VOL. 658], HUMANA PRESS, US, PAGE(S) 167 - 185, ISBN: 978-1-60761-779-2, XP008175975

## Description

### INTRODUCTION

The teachings herein relate to operating a separation coupled tandem mass spectrometer system. More specifically, systems and methods are provided to trigger a group of multiple reaction monitoring (MRM) transitions to reduce retention time variability and adjust the separation for that group to reduce the effects of interfering ions.

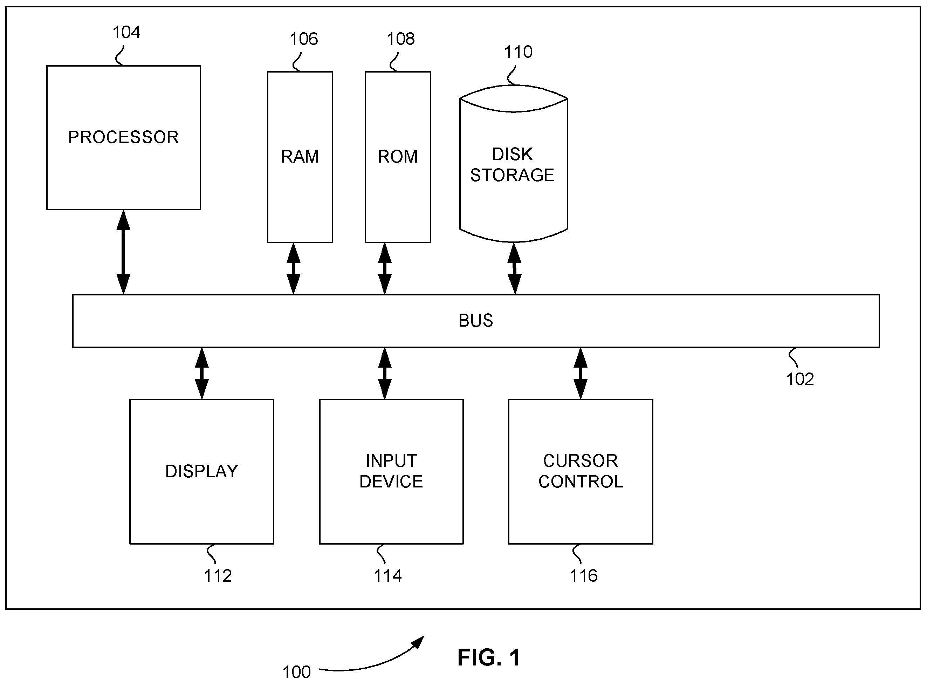

The systems and methods herein can be performed in conjunction with a processor, controller, or computer system, such as the computer system of Figure 1.

### Targeted Protein Assays and Quantified Protein Markers

Currently targeted protein assays are performed, for example, by analyzing tryptic peptides associated with the protein(s) of interest using an MRM workflow. Even though a conventional MRM workflow provides advantages such as improved signal-to-noise (S/N) and a low limit of quantification (LOQ) over other methods for large sets of MRM assays, it suffers from several fundamental issues.

For example, a conventional MRM workflow requires reproducibility of liquid chromatography (LC) conditions, needs to be "fine-tuned" if transferred to other instruments or laboratories, and cannot change gradient conditions even if the LC column stays the same. This causes difficulty in transferring LC methods between labs and puts a burden on the robustness of the instrumentation (LC and columns) and the method itself. In a clinical laboratory, this becomes a problem when transferring and implementing highly multiplexed assays. Note that LOQ is the lowest concentration that can be detected accurately and with precision.

In addition, in the clinical laboratory, there is a need to quantify protein(s) as markers of diseases. The current approach is based on the stable isotope standards and capture by anti-peptide antibodies (SISCAPA) workflow. In this workflow, specific peptides associated with the targeted protein biomarker(s) are affinity-purified and then quantified using a calibration curve generated using the synthetic version of the peptide. The peptides are enriched using antibodies and monitored using the MRM workflow. The SISCAPA workflow is described further below.

One problem with the SISCAPA workflow is that it requires developing antibodies to the peptides of interest. This is a costly and time-consuming step, and, in some cases, it is non-specific and can also impact LOQ if the capture efficiency of the antibody towards the selected peptide is low. In addition, the MRM workflow also suffers from the issues highlighted above (large MRM panel management from LC perspective).

Finally, there is also a need in clinical and toxicology laboratories to screen for large drug panels (drug compounds) and for diagnostic purposes to quantify the drug compounds found. Currently, routine liquid chromatography coupled mass spectrometry/mass spectrometry (LC-MS/MS) methods employ traditional MRM workflows and this requires consistency in the LC method and retention times of the eluting analytes in the panel. Again, this consistency can be lost if transferred to other instruments or laboratories.

As a result, there is a need in workflows targeting protein assays, disease markers, and drug compounds, to maintain consistency in the LC method among instruments or laboratories and to remove the reliance on costly and time-consuming enrichment processes.

### SISCAPA Workflow

U.S. Patent Number 7,632,686 (hereinafter the "'686 Patent") describes the SISCAPA workflow. This workflow is a flow-through method for determining the amount of, or quantitating, target proteins in a sample. An antibody preparation (whether polyclonal or monoclonal or any equivalent specific binding agent) is used to capture and thus enrich both a specific monitored peptide (a specific peptide fragment of a protein to be quantitated in a proteolytic digest of a complex protein sample) and an internal standard peptide (the same chemical structure but including stable isotope labels).

In other words, after a mixture of sample proteins spiked with synthetic stable isotopes labeled peptides (SIS) is digested using trypsin, both the resulting sample peptide and corresponding SIS peptide are enriched using an anti-peptide antibody targeting the sample peptide sequence. All unbound peptides are washed away. Upon elution into and mass analysis by a suitable mass spectrometer (LC-MS/MS), the sample peptide and corresponding SIS peptide are quantitated. Their measured abundance ratio is used to calculate the abundance of the sample peptide, and its parent protein, in the initial sample.

### Sentinel Analysis

MRM or selected reaction monitoring (SRM) is a targeted acquisition method, as described below. In MRM, one or more transitions of a precursor ion to a product ion are predefined for compounds of a sample. As the sample is being introduced into the tandem mass spectrometer, the precursor ion of each transition of the one or more transitions is selected and fragmented and the product ion of each transition is mass analyzed, producing a product ion intensity for each transition.

MRM is often performed in liquid chromatography coupled mass spectrometry/mass spectrometry (LC-MS/MS) experiments that are used to identify or quantify one or more compounds of interest. When a complex sample that includes many different compounds of interest is analyzed, the number of MRM transitions used in the analysis may become large. In order to reduce the number of MRM transitions that are performed in one cycle of a tandem mass spectrometer, a method for scheduling the MRM transitions was developed. This method is referred to as scheduled MRM.

In scheduled MRM, each MRM transition to be analyzed during the experiment is also assigned a retention time or retention time range. During the experiment, MRM transitions are then added to and removed from a list of transitions to be executed during each cycle of the tandem mass spectrometer based on their retention time or retention time range. In this way, the number of transitions being executed during any one cycle is reduced.

Unfortunately, however, in some instances, compounds of interest may not separate from a sample at the retention times specified in a scheduled MRM experiment. For example, the scheduled MRM experiment may be performed by a different laboratory or under different experimental conditions. In addition, scheduled MRM is dependent on the accuracy and absolute value of the retention time used for each transition. Whenever the separation device changes or the gradient of separation changes, the retention time for each transition must be recomputed. This becomes particularly cumbersome when workflows include thousands of MRM transitions. This also makes it difficult to use scheduled MRM workflows across separation devices produced by different manufacturers that have different elution rates. Further, the separation may not be based on retention time at all.

As a result, a method for triggering MRM transitions that is not based on retention time was developed. In this method, a scout or sentinel MRM transition is used to trigger a group of additional MRM transitions to be analyzed. More specifically, the MRM transitions of an experiment for a sample are divided into two or more contiguous groups of MRM transitions so that the groups are executed sequentially. Each group includes at least one scout or sentinel MRM transition that identifies the next group of MRM transitions to be executed.

During acquisition, a first group of MRM transitions is selected for monitoring. When at least one sentinel MS/MS scan in the first group is detected by the tandem mass spectrometer, the next group of MRM transitions identified by the at least one sentinel MS/MS scan is added to the list of transitions monitored by the tandem mass spectrometer. In other words, at least one sentinel MS/MS scan in each group is used to trigger the transitions in the next contiguous group.

A group of MRM transitions can also be removed from monitoring. For example, once at least one sentinel MS/MS scan in the next contiguous group is detected, the transitions in the first group can be removed from monitoring.

As a result, by using sentinel transitions to trigger the addition and subtraction of MRM transitions from monitoring, the overall number of MRM transitions being monitored at any one time is reduced. In addition, because the groups of transitions are not dependent on a specific retention time, workflows based on these systems and methods can be used without modification whenever the separation device changes or the gradient of separation changes.

U.S. Patent Number 10,566,178 (hereinafter the "'178 Patent") describes using sentinel transitions to overcome the limitations of scheduled MRM. The '178 Patent describes systems and methods in which sentinel transitions are used in conjunction with a system that includes a separation device, such as LC, for separating compounds from a sample.

U.S. Patent Application Number 16/790,803 (hereinafter the "'803 Application") is a continuation application of the '178 Patent and describes systems and methods in which sentinel transitions are used without a separation device. The '803 Application essentially describes systems and methods in which sentinel transitions are used in conjunction with any method of introducing compounds of interest into a tandem mass spectrometer.

One exemplary method of introducing compounds of interest into a tandem mass spectrometer without a separation device is through the use of a sample introduction device. U.S. Provisional Patent Application Number 63/029,226 (hereinafter the "'226 Application") describes systems and methods in which scout or sentinel transitions are used in conjunction with a sample introduction device that ejects samples at an ejection time and according to a sample order. An exemplary sample introduction device that ejects samples at an ejection time and according to a sample order is an acoustic droplet ejection (ADE) device that delivers samples rapidly to an open port interface (OPI) from individual microtiter plate wells.

MRM experiments are typically performed using "low resolution" instruments that include, but are not limited to, triple quadrupole (QqQ) or quadrupole linear ion trap (QqLIT) devices. With the advent of "high resolution" instruments, there was a desire to collect MS and MS/MS using workflows that are similar to QqQ/QqLIT systems. High resolution instruments include, but are not limited to, quadrupole time-of-flight (QqTOF) or orbitrap devices. These high-resolution instruments also provide new functionality.

MRM on QqQ/QqLIT systems is the standard mass spectrometric technique of choice for targeted quantification in all application areas, due to its ability to provide the highest specificity and sensitivity for the detection of specific components in complex mixtures. However, the speed and sensitivity of today's accurate mass systems have enabled a new quantification strategy with similar performance characteristics. In this strategy (termed MRM high resolution (MRM-HR) or parallel reaction monitoring (PRM)), looped MS/MS spectra are collected at high-resolution with short accumulation times, and then fragment ions (product ions) are extracted post-acquisition to generate MRM-like peaks for integration and quantification. With instrumentation like the TRIPLETOF^{®} Systems of AB SCIEX^{™}, this targeted technique is sensitive and fast enough to enable quantitative performance similar to higher end triple quadrupole instruments, with full fragmentation data measured at high resolution and high mass accuracy.

In other words, in methods such as MRM-HR, a high-resolution precursor ion mass spectrum is obtained, one or more precursor ions are selected and fragmented, and a high-resolution full product ion spectrum is obtained for each selected precursor ion. A full product ion spectrum is collected for each selected precursor ion but a product ion mass of interest can be specified and everything other than the mass window of the product ion mass of interest can be discarded.

As a result, scout or sentinel analysis can similarly be performed with a high-resolution tandem mass spectrometer capable of performing MRM-HR. In the conventional method of performing sentinel analysis described above, the detection of the product ion of an MRM transition provides the selectivity needed to avoid a false trigger of additional associated MRM transitions. However, the added functionally of high-resolution tandem mass spectrometers provides the needed selectivity in other ways.

U.S. Provisional Patent Application Number 63/088,669 (hereinafter the "'669 Application") describes systems and methods in which an accurate mass of a precursor ion detected during a high resolution MS scan is used to trigger a group of high resolution precursor ion to full product ion spectrum MS/MS scans in sentinel analysis. Essentially, a high resolution MS scan can produce a selectivity similar to an MRM transition in conventional sentinel analysis.

The '669 Application also describes systems and methods in which an isotopic pattern of a precursor ion detected during a high resolution MS scan is further used to trigger a group of high resolution precursor ion to full product ion spectrum MS/MS scans in sentinel analysis. The isotopic pattern is used in addition to the accurate mass of the precursor ion to provide the selectivity for sentinel analysis.

### Mass Spectrometry Background

Mass spectrometers are often coupled with separation devices, such as chromatography devices, or sample introduction systems, such as an ADE device and OPI, in order to identify and characterize compounds of interest from a sample or to analyze multiple samples. In such a coupled system, the eluting or injected solvent is ionized and a series of mass spectra are obtained from the eluting solvent at specified time intervals called retention times. These retention times range from, for example, 1 second to 100 minutes or greater. The series of mass spectra form a chromatogram, or extracted ion chromatogram (XIC).

Peaks found in the XIC are used to identify or characterize a known peptide or compound in a sample, for example. More particularly, the retention times of peaks and/or the area of peaks are used to identify or characterize (quantify) a known peptide or compound in the sample. In the case of multiple samples provided over time by a sample introduction device, the retention times of peaks are used to align the peaks with the correct sample.

In traditional separation coupled mass spectrometry systems, a fragment or product ion of a known compound is selected for analysis. A tandem mass spectrometry or mass spectrometry/mass spectrometry (MS/MS) scan is then performed at each interval of the separation for a mass range that includes the product ion. The intensity of the product ion found in each MS/MS scan is collected over time and analyzed as a collection of spectra, or an XIC, for example.

In general, tandem mass spectrometry, or MS/MS, is a well-known technique for analyzing compounds. Tandem mass spectrometry involves ionization of one or more compounds from a sample, selection of one or more precursor ions of the one or more compounds, fragmentation of the one or more precursor ions into fragment or product ions, and mass analysis of the product ions.

Tandem mass spectrometry can provide both qualitative and quantitative information. The product ion spectrum can be used to identify a molecule of interest. The intensity of one or more product ions can be used to quantitate the amount of the compound present in a sample.

A large number of different types of experimental methods or workflows can be performed using a tandem mass spectrometer. Three broad categories of these workflows are targeted acquisition, information dependent acquisition (IDA) or data-dependent acquisition (DDA), and data-independent acquisition (DIA).

In a targeted acquisition method, one or more transitions of a precursor ion to a product ion are predefined for a compound of interest. As a sample is being introduced into the tandem mass spectrometer, the one or more transitions are interrogated or monitored during each time period or cycle of a plurality of time periods or cycles. In other words, the mass spectrometer selects and fragments the precursor ion of each transition and performs a targeted mass analysis only for the product ion of the transition. As a result, an intensity (a product ion intensity) is produced for each transition. Targeted acquisition methods include, but are not limited to, MRM and selected reaction monitoring (SRM).

In a targeted acquisition method, a list of transitions is typically interrogated during each cycle time. In order to decrease the number of transitions that are interrogated at any one time, some targeted acquisition methods have been modified to include a retention time or a retention time range for each transition. Only at that retention time or within that retention time range will that particular transition be interrogated. One targeted acquisition method that allows retention times to be specified with transitions is referred to as scheduled MRM.

In an IDA method, a user can specify criteria for performing an untargeted mass analysis of product ions, while a sample is being introduced into the tandem mass spectrometer. For example, in an IDA method, a precursor ion or mass spectrometry (MS) survey scan is performed to generate a precursor ion peak list. The user can select criteria to filter the peak list for a subset of the precursor ions on the peak list. MS/MS is then performed on each precursor ion of the subset of precursor ions. A product ion spectrum is produced for each precursor ion. MS/MS is repeatedly performed on the precursor ions of the subset of precursor ions as the sample is being introduced into the tandem mass spectrometer.

In proteomics and many other sample types, however, the complexity and dynamic range of compounds are very large. This poses challenges for traditional targeted and IDA methods, requiring very high-speed MS/MS acquisition to deeply interrogate the sample in order to both identify and quantify a broad range of analytes.

As a result, DIA methods, the third broad category of tandem mass spectrometry, were developed. These DIA methods have been used to increase the reproducibility and comprehensiveness of data collection from complex samples. DIA methods can also be called non-specific fragmentation methods. In a traditional DIA method, the actions of the tandem mass spectrometer are not varied among MS/MS scans based on data acquired in a previous precursor or product ion scan. Instead, a precursor ion mass range is selected. A precursor ion mass selection window is then stepped across the precursor ion mass range. All precursor ions in the precursor ion mass selection window are fragmented and all of the product ions of all of the precursor ions in the precursor ion mass selection window are mass analyzed.

The precursor ion mass selection window used to scan the mass range can be very narrow so that the likelihood of multiple precursors within the window is small. This type of DIA method is called, for example, MS/MS^{ALL}. **In an** MS/MS^{ALL} method, a precursor ion mass selection window of about 1 amu is scanned or stepped across an entire mass range. A product ion spectrum is produced for each 1 amu precursor mass window. The time it takes to analyze or scan the entire mass range once is referred to as one scan cycle. Scanning a narrow precursor ion mass selection window across a wide precursor ion mass range during each cycle, however, is not practical for some instruments and experiments.

As a result, a larger precursor ion mass selection window, or selection window with a greater width, is stepped across the entire precursor mass range. This type of DIA method is called, for example, SWATH acquisition. In a SWATH acquisition, the precursor ion mass selection window stepped across the precursor mass range in each cycle may have a width of 5-25 amu, or even larger. Like the MS/MS^{ALL} method, all the precursor ions in each precursor ion mass selection window are fragmented, and all of the product ions of all of the precursor ions in each mass selection window are mass analyzed.

### SUMMARY

The invention is defined by the independent claims. Further aspects and preferred embodiments are defined in the dependent claims. Aspects, embodiments and examples of the present disclosure which do not fall under the scope of the appended claims do not form part of the invention and are merely provided for illustrative purposes. A system, method, and computer program product are provided for triggering a group of MRM transitions and adjusting the separation for that group. The system includes a separation device, an ion source, a tandem mass spectrometer, and a processor.

The separation device separates one or more known compounds from a sample mixture. The separation device also allows processor-controlled adjustment of at least one parameter of the separation device during the separation. The ion source ionizes the separated one or more compounds received from the separation device, producing an ion beam of one or more precursor ions.

The tandem mass spectrometer receives the ion beam from the ion source. For each cycle of a plurality of cycles, the tandem mass spectrometer tandem executes on the ion beam a series of MRM precursor ion to product ion transitions read from a list. For each transition of the list, the tandem mass spectrometer selects and fragments a precursor ion of the transition and mass analyzes a product ion of the transition.

The processor receives two or more contiguous groups of MRM transitions for monitoring the one or more known compounds. Each group of the two or more contiguous groups is monitored separately by the tandem mass spectrometer during the plurality of cycles and includes at least one sentinel transition. The at least one sentinel transition in each group identifies a next group of the two or more contiguous groups that is to be monitored and identifies a value for the at least one parameter for the next group.

The processor places a first group of the two or more contiguous groups on the list of the tandem mass spectrometer. When at least one sentinel transition of the first group is detected by the tandem mass spectrometer, the processor places a next group of the two or more contiguous groups identified by the at least one sentinel transition on the list and adjusts the at least one parameter of the separation device to a value identified by the at least one sentinel transition for the next group.

These and other features of the applicant's teachings are set forth herein.

### BRIEF DESCRIPTION OF THE DRAWINGS

The skilled artisan will understand that the drawings, described below, are for illustration purposes only. The drawings are not intended to limit the scope of the present teachings in any way.
Figure 1 is a block diagram that illustrates a computer system, upon which embodiments of the present teachings may be implemented.
Figure 2 is a schematic diagram of a system for triggering a group of MRM transitions and adjusting the separation for that group, in accordance with various embodiments.
Figure 3 is a flowchart showing a method for triggering a group of MRM transitions and adjusting the separation for that group, in accordance with various embodiments.
Figure 4 is a schematic diagram of a system that includes one or more distinct software modules that perform a method for triggering a group of MRM transitions and adjusting the separation, in accordance with various embodiments.

Before one or more embodiments of the present teachings are described in detail, one skilled in the art will appreciate that the present teachings are not limited in their application to the details of construction, the arrangements of components, and the arrangement of steps set forth in the following detailed description or illustrated in the drawings. Also, it is to be understood that the phraseology and terminology used herein is for the purpose of description and should not be regarded as limiting.

### DESCRIPTION OF VARIOUS EMBODIMENTS

### COMPUTER-IMPLEMENTED SYSTEM

Figure 1 is a block diagram that illustrates a computer system 100, upon which embodiments of the present teachings may be implemented. Computer system 100 includes a bus 102 or other communication mechanism for communicating information, and a processor 104 coupled with bus 102 for processing information. Computer system 100 also includes a memory 106, which can be a random-access memory (RAM) or other dynamic storage device, coupled to bus 102 for storing instructions to be executed by processor 104. Memory 106 also may be used for storing temporary variables or other intermediate information during execution of instructions to be executed by processor 104. Computer system 100 further includes a read only memory (ROM) 108 or other static storage device coupled to bus 102 for storing static information and instructions for processor 104. A storage device 110, such as a magnetic disk or optical disk, is provided and coupled to bus 102 for storing information and instructions.

Computer system 100 may be coupled via bus 102 to a display 112, such as a cathode ray tube (CRT) or liquid crystal display (LCD), for displaying information to a computer user. An input device 114, including alphanumeric and other keys, is coupled to bus 102 for communicating information and command selections to processor 104. Another type of user input device is cursor control 116, such as a mouse, a trackball or cursor direction keys for communicating direction information and command selections to processor 104 and for controlling cursor movement on display 112. This input device typically has two degrees of freedom in two axes, a first axis *(i.e.,* x) and a second axis *(i.e.,* y), that allows the device to specify positions in a plane.

A computer system 100 can perform the present teachings. Consistent with certain implementations of the present teachings, results are provided by computer system 100 in response to processor 104 executing one or more sequences of one or more instructions contained in memory 106. Such instructions may be read into memory 106 from another computer-readable medium, such as storage device 110. Execution of the sequences of instructions contained in memory 106 causes processor 104 to perform the process described herein. Alternatively hard-wired circuitry may be used in place of or in combination with software instructions to implement the present teachings. Thus, implementations of the present teachings are not limited to any specific combination of hardware circuitry and software.

In various embodiments, computer system 100 can be connected to one or more other computer systems, like computer system 100, across a network to form a networked system. The network can include a private network or a public network such as the Internet. In the networked system, one or more computer systems can store and serve the data to other computer systems. The one or more computer systems that store and serve the data can be referred to as servers or the cloud, in a cloud computing scenario. The one or more computer systems can include one or more web servers, for example. The other computer systems that send and receive data to and from the servers or the cloud can be referred to as client or cloud devices, for example.

The term "computer-readable medium" as used herein refers to any media that participates in providing instructions to processor 104 for execution. Such a medium may take many forms, including but not limited to, non-volatile media, volatile media, and transmission media. Non-volatile media includes, for example, optical or magnetic disks, such as storage device 110. Volatile media includes dynamic memory, such as memory 106. Transmission media includes coaxial cables, copper wire, and fiber optics, including the wires that comprise bus 102.

Common forms of computer-readable media or computer program products include, for example, a floppy disk, a flexible disk, hard disk, magnetic tape, or any other magnetic medium, a CD-ROM, digital video disc (DVD), a Blu-ray Disc, any other optical medium, a thumb drive, a memory card, a RAM, PROM, and EPROM, a FLASH-EPROM, any other memory chip or cartridge, or any other tangible medium from which a computer can read.

Various forms of computer readable media may be involved in carrying one or more sequences of one or more instructions to processor 104 for execution. For example, the instructions may initially be carried on the magnetic disk of a remote computer. The remote computer can load the instructions into its dynamic memory and send the instructions over a telephone line using a modem. A modem local to computer system 100 can receive the data on the telephone line and use an infra-red transmitter to convert the data to an infra-red signal. An infra-red detector coupled to bus 102 can receive the data carried in the infra-red signal and place the data on bus 102. Bus 102 carries the data to memory 106, from which processor 104 retrieves and executes the instructions. The instructions received by memory 106 may optionally be stored on storage device 110 either before or after execution by processor 104.

In accordance with various embodiments, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

The following descriptions of various implementations of the present teachings have been presented for purposes of illustration and description. It is not exhaustive and does not limit the present teachings to the precise form disclosed. Modifications and variations are possible in light of the above teachings or may be acquired from practicing of the present teachings. Additionally, the described implementation includes software but the present teachings may be implemented as a combination of hardware and software or in hardware alone. The present teachings may be implemented with both object-oriented and non-object-oriented programming systems.

### SENTINEL TRIGGERED MRM GROUP AND SEPARATION

As described above, currently targeted protein assays are performed, for example, by analyzing tryptic peptides associated with the protein(s) of interest using an MRM workflow. However, a conventional MRM workflow requires reproducibility of LC conditions, needs to be "fine-tuned" if transferred to other instruments or laboratories, and cannot change gradient conditions even if the LC column stays the same. This causes difficulty in transferring LC methods between labs and puts a burden on the robustness of the instrumentation (LC and columns) and the method itself.

In addition, in the clinical laboratory, there is a need to quantify protein(s) as markers of diseases. The current approach is based on the stable isotope standards and capture by anti-peptide antibodies (SISCAPA) workflow. One problem with the SISCAPA workflow is that it requires developing antibodies to the peptides of interest. This is a costly and time-consuming step. In addition, the MRM workflow also suffers from the issues highlighted above.

Finally, there is also a need in clinical and toxicology laboratories to screen for large drug panels (drug compounds) and for diagnostic purposes to quantify the drug compounds found. Currently, routine LC-MS/MS methods employ traditional MRM workflows and this requires consistency in the LC method and retention times of the eluting analytes in the panel. Again, this consistency can be lost if transferred to other instruments or laboratories.

As a result, there is a need in workflows targeting protein assays, disease markers, and drug compounds, to maintain consistency in the LC method among instruments or laboratories and to remove the reliance on costly and time-consuming enrichment processes.

In various embodiments, a scout or sentinel MRM transition is used in peptide or small molecule quantitation to maintain consistency in the LC method among instruments or laboratories. Sentinel MRM provides transparency to retention time shift and can be immune to changes in the LC gradient, changes in the pump configuration, and changes in LC columns. In addition, Sentinel MRM also ensures easier incorporation of new analytes or species to screen and provides a far simpler way to manage large panels of MRMs and ensure data collection than techniques such as SISCAPA. These advantages of sentinel MRM have been demonstrated in qualitative analyses with pesticide panels and some peptide panels. These advantages of sentinel MRM have also been predicted for qualitative analyses with drugs of abuse panels.

In various embodiments, the use of a sentinel MRM workflow provides an improvement over the conventional SISCAPA workflow in the quantitative analysis of compounds such as peptides and small molecules. For example, protein marker(s) of interest can be identified using sentinel MRMs selected from early eluting peptide(s) specific to the marker(s) followed by identifying additional peptides(s) specific to the protein or panels. In other words, large protein panels are screened systematically, but the sentinel MRM workflow more quickly focuses on the limited number of proteins or small molecules that are relevant to the sample supplied (only subset pertaining to disease would be present for example). Monitoring multiple peptides to a protein panel or multiple compounds to a small molecule compound panel provides confidence in the identification of the analyte(s) of interest.

In various embodiments, quantitation is performed by adding known concentrations of isotopically labeled versions of sentinel compounds to the sample that serve as internal standards or as a single point calibration. In other words, quantitation of the analyte of interest is performed using an isotopically labeled version of the sentinel analyte (peptide or small molecule) spiked in the sample at a predetermined concentration. The method is then set up for the screening of large panels (e.g., drug panel, cancer panel, etc.) or as a diagnostics assay. The sentinel analyte (peptide or small molecule) spiked in the sample at predetermined concentrations can be used to generate a calibration curve that is used to calculate the quantification of the analyte of interest.

In various embodiments, the sentinel MRM workflow solves the problem caused by small variations in retention time for peptides and small molecules analyzed by LC-MS/MS in quantifying the amount of peptide (and associated protein) or small molecule present in a given experiment. Small changes in retention time can lead to inaccurate or missing quantification results and are exacerbated when sample complexity is high.

The conventional SISCAPA workflow reduces the likelihood of inaccurate quantification by reducing the complexity of the sample before analysis through antibody purification of selected peptides. However, the production of antibodies required for SISCAPA is time-consuming and expensive. Various embodiments described herein make it possible to compensate for retention time variability without the need to perform anti-body purification. However, the antibody purification of SISCAPA can also reduce the effects of ion suppression and interferences in the quantitation.

In various embodiments, the sentinel MRM workflow further uses a sentinel transition to trigger a change in a parameter applied to a sample separation device to reduce the effects of ion suppression and interferences. This sentinel-triggered change to the sample separation removes the reliance on costly and time-consuming enrichment processes like the antibody approach of SISCAPA. For example, in addition to identifying the presence of an analyte of interest, a sentinel transition can trigger an increase in the LC gradient time to extend the chromatography for an analyte known to be susceptible to inferences. Increasing the gradient time increases the retention time between an LC peak of the analyte of interest and an interfering LC peak.

The LC gradient is the solid composition of the eluting mixture over time. A typical LC gradient is, for example, a transition from 5% (aqueous) to 95% (organic) solid composition in five minutes. Increasing the gradient time increases the time for the solid composition transition, which, in turn, increases the time between LC peaks. Increasing the time between LC peaks reduces the effects of ion suppression and interferences.

**In** various embodiments, the LC gradient time is varied by computer-controlled adjustment of a proportioning valve between at least two LC solvents. These two solvents can be an aqueous solvent and an organic solvent, for example.

Although the parameter applied to a sample separation device has just been described as a change to the gradient time of an LC separation, any parameter of any type of sample separation can be triggered by a sentinel transition to reduce the effects of ion suppression and interferences. A separation device can separate compounds over time using one of a variety of techniques. These techniques include, but are not limited to, ion mobility, gas chromatography (GC), or capillary electrophoresis (CE) in addition to LC.

### System for triggering MRM group and adjusting separation

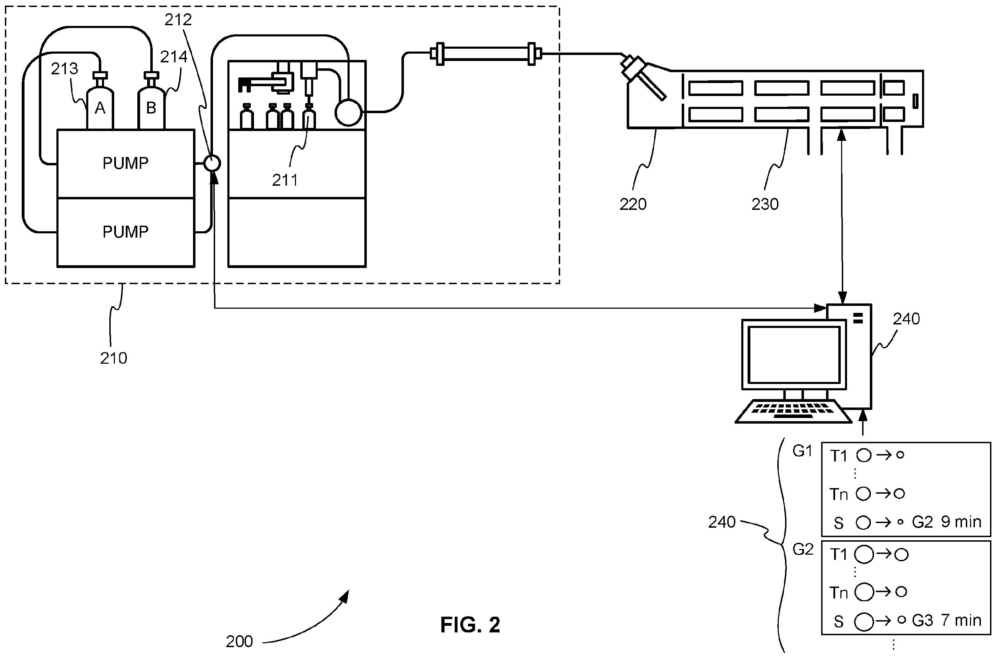

Figure 2 is a schematic diagram of a system 200 for triggering a group of MRM transitions and adjusting the separation for that group, in accordance with various embodiments. As described above, triggering a group of MRM transitions reduces retention time variability. Adjusting the separation for that group reduces interferences. System 200 includes separation device 210, ion source 220, tandem mass spectrometer 230, and processor 240.

Separation device 210 separates one or more known compounds from a sample mixture 211. Separation device 210 also allows processor-controlled adjustment of at least one parameter of separation device 210 during the separation. Separation device 210 can separate compounds over time using one of a variety of techniques. These techniques include, but are not limited to, ion mobility, gas chromatography (GC), liquid chromatography (LC), capillary electrophoresis (CE), or flow injection analysis (FIA).

Ion source 220 can be part of tandem mass spectrometer 230 or can be a separate device. Ion source 220 ionizes the separated one or more compounds received from separation device 210, producing an ion beam of one or more precursor ions.

Tandem mass spectrometer 230 can include, for example, one or more physical mass filters and one or more physical mass analyzers. A mass analyzer of tandem mass spectrometer 230 can include, but is not limited to, a quadrupole, a time-of-flight (TOF) mass analyzer, an ion trap, an orbitrap, or a Fourier transform mass analyzer.

Tandem mass spectrometer 230 receives the ion beam from ion source 220. For each cycle of a plurality of cycles, tandem mass spectrometer 230 executes on the ion beam a series of MRM precursor ion to product ion transitions read from a list. For each transition of the list, tandem mass spectrometer 230 selects and fragments a precursor ion of the transition and mass analyzes a product ion of the transition.

Processor 240 can be, but is not limited to, a computer, microprocessor, or any device capable of sending and receiving control signals and data from tandem mass spectrometer 230 and processing data. Processor 240 can be, for example, computer system 100 of Figure 1. In various embodiments, processor 240 is in communication with tandem mass spectrometer 230 and separation device 210.

Processor 240 receives two or more contiguous groups 241 of MRM transitions for monitoring the one or more known compounds. Two or more contiguous groups 241 can, for example, be provided as input from a user for a particular experiment or method. Two or more contiguous groups 241 can include transitions for known compounds such as those of a pesticide panel, a peptide panel, or a drugs of abuse panel, for example. Each group may correspond to a separate protein, peptide, or small molecule.

Each group of two or more contiguous groups 241 is monitored separately by tandem mass spectrometer 230 during the plurality of cycles and includes at least one sentinel transition. The at least one sentinel transition in each group identifies a next group of two or more contiguous groups 241 that is to be monitored and identifies a value for the at least one parameter for the next group.

Processor 240 places a first group of two or more contiguous groups 241 on the list of tandem mass spectrometer 230. When at least one sentinel transition of the first group is detected by tandem mass spectrometer 230, processor 240 places a next group of two or more contiguous groups 241 identified by the at least one sentinel transition on the list and adjusts the at least one parameter of the separation device to a value identified by the at least one sentinel transition for the next group.

In various embodiments, the one or more known compounds can include one or more known peptides tryptically digested from one or more proteins or one or more known small molecules. In various embodiments, the one or more small molecules can include pesticides or drugs of abuse.

In various embodiments, the system of Figure 2 can be used for the quantitation. For example, the one or more known compounds include at least one known compound of mixture 211. The one or more known compounds also include a corresponding isotopically labeled version of the at least one known compound that has been added to mixture 211 in a known concentration to act as a standard for quantitation.

In various embodiments, separation device 210 includes an LC device, and the at least one parameter comprises an LC gradient time of the separation.

In various embodiments, the next group includes a transition for the at least one known compound of mixture 211 and a transition for the isotopically labeled version of the at least one known compound. The value identified by the at least one sentinel transition for the LC gradient time of the separation is based on a predetermined probability that the mixture includes an interference with the at least one known compound.

In various embodiments, if the predetermined probability is high, the value increases the LC gradient time. Alternatively, if the predetermined probability is low, the value decreases the LC gradient time.

In various embodiments, processor 240 adjusts the LC gradient time by adjusting a proportional valve 212 between solvents. The solvents include two solvents, for example. The two solvents include an aqueous solvent 213 and an organic solvent 214, for example.

In various embodiments, tandem mass spectrometer 230 further detects compound intensities for the transition for the at least one known compound and standard intensities for the transition for the isotopically labeled version of the at least one known compound for one or more cycles of the plurality of cycles. Processor 240 further calculates a quantitative value for the at least one known compound from the detected compound intensities, detected standard intensities, and the known concentration.

### Method for triggering MRM group and adjusting separation

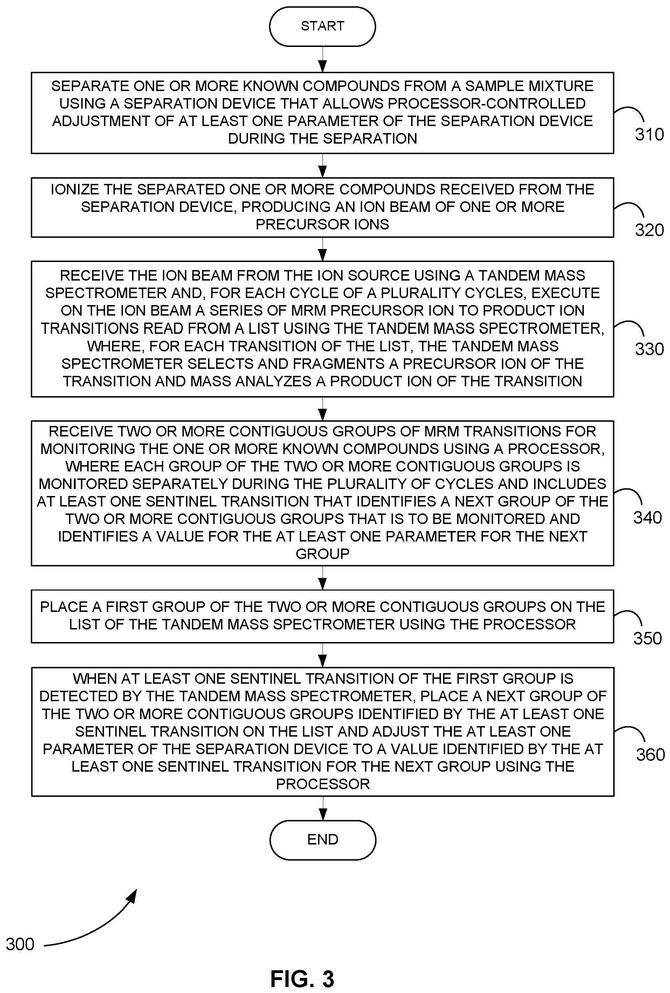

Figure 3 is a flowchart showing a method 300 for triggering a group of MRM transitions and adjusting the separation for that group, in accordance with various embodiments.

In step 310 of method 300, one or more known compounds are separated from a sample mixture using a separation device that allows processor-controlled adjustment of at least one parameter of the separation device during the separation.

In step 320, the separated one or more compounds received from the separation device are ionized, producing an ion beam of one or more precursor ions.

In step 330, the ion beam is received from the ion source using a tandem mass spectrometer. For each cycle of a plurality of cycles, the tandem mass spectrometer executes on the ion beam a series of MRM precursor ion to product ion transitions read from a list. For each transition of the list, the tandem mass spectrometer selects and fragments a precursor ion of the transition and mass analyzes a product ion of the transition.

In step 340, two or more contiguous groups of MRM transitions for monitoring the one or more known compounds are received using a processor. Each group of the two or more contiguous groups is monitored separately during the plurality of cycles. Each group includes at least one sentinel transition that identifies a next group of the two or more contiguous groups that is to be monitored and identifies a value for the at least one parameter for the next group.

In step 350, a first group of the two or more contiguous groups is placed on the list of the tandem mass spectrometer using the processor.

In step 360, when at least one sentinel transition of the first group is detected by the tandem mass spectrometer, a next group of the two or more contiguous groups identified by the at least one sentinel transition is placed on the list using the processor. In addition, the at least one parameter of the separation device is adjusted to a value identified by the at least one sentinel transition for the next group using the processor.

### Computer program product for triggering MRM group and adjusting separation

In various embodiments, computer program products include a tangible computer-readable storage medium whose contents include a program with instructions being executed on a processor so as to perform a method for triggering a group of MRM transitions and adjusting the separation. This method is performed by a system that includes one or more distinct software modules.

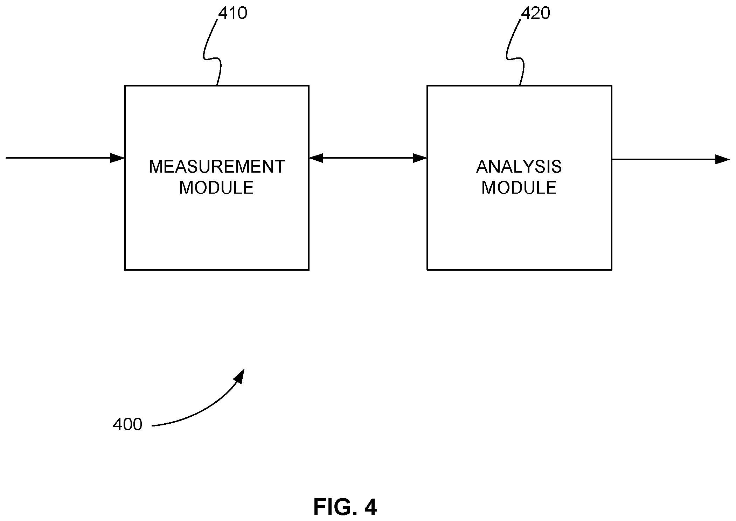

Figure 4 is a schematic diagram of a system 400 that includes one or more distinct software modules that perform a method for triggering a group of MRM transitions and adjusting the separation, in accordance with various embodiments. System 400 includes measurement module 410 and analysis module 420.

For each cycle of a plurality of cycles, measurement module 410 instructs a tandem mass spectrometer to execute on an ion beam a series of MRM precursor ion to product ion transitions read from a list. For each transition of the list, the tandem mass spectrometer selects and fragments a precursor ion of the transition and mass analyzes a product ion of the transition. The ion beam is produced by an ion source that ionizes one or more compounds separated from a sample mixture. The one or more compounds are separated using a separation device that allows processor-controlled adjustment of at least one parameter of the separation device during the separation.

Analysis module 420 receives two or more contiguous groups of MRM transitions for monitoring the one or more known compounds. Each group of the two or more contiguous groups is monitored separately during the plurality of cycles. Each group includes at least one sentinel transition that identifies a next group of the two or more contiguous groups that is to be monitored and identifies a value for the at least one parameter for the next group.

Analysis module 420 places a first group of the two or more contiguous groups on the list of the tandem mass spectrometer. When at least one sentinel transition of the first group is detected by the tandem mass spectrometer, analysis module 420 places a next group of the two or more contiguous groups identified by the at least one sentinel transition on the list. Analysis module 420 also adjusts the at least one parameter of the separation device to a value identified by the at least one sentinel transition for the next group.

While the present teachings are described in conjunction with various embodiments, it is not intended that the present teachings be limited to such embodiments. On the contrary, the present teachings encompass various alternatives, modifications, and equivalents, as will be appreciated by those of skill in the art.

Further, in describing various embodiments, the specification may have presented a method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. Similarly, though the described application used MRM as a detection technique, the described method can be applied to any targeted analysis for MS/MS analysis such as MRM3, single ion monitoring (SIM) or even targeted product ion scan (TOF-MS). In addition, the claims directed to the method and/or process should not be limited to the performance of their steps in the order written, and one skilled in the art can readily appreciate that the sequences may be varied and still remain within the scope of the claims.

## Claims

1. A system (200) for triggering a group of multiple reaction monitoring (MRM) transitions and adjusting the separation for that group, comprising:
a separation device (201) that separates (310) one or more known compounds from a sample mixture and allows processor-controlled adjustment of at least one parameter of the separation device during the separation;
an ion source (220) that ionizes (320) the separated one or more compounds received from the separation device, producing an ion beam of one or more precursor ions;
a tandem mass spectrometer (230) that receives (330) the ion beam from the ion source and for each cycle of a plurality of cycles executes on the ion beam a series of MRM precursor ion to product ion transitions read from a list, wherein for each transition of the list, the tandem mass spectrometer selects and fragments a precursor ion of the transition and mass analyzes a product ion of the transition; and
a processor (240) in communication with the tandem mass spectrometer that
receives (340) two or more contiguous groups of MRM transitions for monitoring the one or more known compounds, wherein each group of the two or more contiguous groups is monitored separately during the plurality of cycles and includes at least one sentinel transition that identifies a next group of the two or more contiguous groups that is to be monitored and identifies a value for the at least one parameter for the next group,
places (350) a first group of the two or more contiguous groups on the list of the tandem mass spectrometer, and
when at least one sentinel transition of the first group is detected by the tandem mass spectrometer, places (360) a next group of the two or more contiguous groups identified by the at least one sentinel transition on the list and adjusts the at least one parameter of the separation device to a value identified by the at least one sentinel transition for the next group.

2. The system of claim **1,** wherein the one or more known compounds comprise one or more known peptides typically digested from one or more proteins.

3. The system of claim **1,** wherein the one or more known compounds comprise one or more known small molecules.

4. The system of claim **1,** wherein the one or more small molecules comprise pesticides or drugs of abuse.

5. The system of claim **1,** wherein the one or more known compounds include at least one known compound of the mixture and a corresponding isotopically labeled version of the at least one known compound added to the mixture in a known concentration to act as a standard for quantitation.

6. The system of claim 5, wherein the separation device comprises a liquid chromatography (LC) device and the at least one parameter comprises an LC gradient time of the separation.

7. The system of claim 6,
wherein the next group includes a transition for the at least one known compound of the mixture and a transition for the isotopically labeled version of the at least one known compound and
wherein the value identified by the at least one sentinel transition for the LC gradient time of the separation is based on a predetermined probability that the mixture includes an interference with the at least one known compound.

8. The system of claim 7, wherein if the predetermined probability is high, the value increases the LC gradient time.

9. The system of claim 7, wherein if the predetermined probability is low, the value decreases the LC gradient time.

10. The system of claim 7, wherein the processor adjusts the LC gradient time by adjusting a proportional valve between solvents.

11. The system of claim 10, wherein the solvents comprise two solvents.

12. The system of claim **11,** wherein two solvents comprise an aqueous solvent and an organic solvent.

13. The system of claim 7,
wherein the tandem mass spectrometer (230) further detects compound intensities for the transition for the at least one known compound and standard intensities for the transition for the isotopically labeled version of the at least one known compound for one or more cycles of the plurality of cycles, and
wherein the system further calculates a quantitative value for the at least one known compound from the detected compound intensities, detected standard intensities, and the known concentration,
optionally wherein the detected standard intensities for the transition for the isotopically labeled version of the at least one known compound are used to generate a calibration curve and the quantitative value for the at least one known compound is calculated using the calibration curve.

14. A method (300) for triggering a group of multiple reaction monitoring (MRM) transitions and adjusting the separation for that group, comprising:
separating (310) one or more known compounds from a sample mixture using a separation device (210) that allows processor-controlled adjustment of at least one parameter of the separation device during the separation;
ionizing (320) the separated one or more compounds received from the separation device, producing an ion beam of one or more precursor ions;
receiving (330) the ion beam from the ion source (220) using a tandem mass spectrometer (230) and, for each cycle of a plurality of cycles, executing on the ion beam a series of MRM precursor ion to product ion transitions read from a list using the tandem mass spectrometer, wherein for each transition of the list, the tandem mass spectrometer selects and fragments a precursor ion of the transition and mass analyzes a product ion of the transition;
receiving (340) two or more contiguous groups of MRM transitions for monitoring the one or more known compounds using a processor (240), wherein each group of the two or more contiguous groups is monitored separately during the plurality of cycles and includes at least one sentinel transition that identifies a next group of the two or more contiguous groups that is to be monitored and identifies a value for the at least one parameter for the next group;
placing (350) a first group of the two or more contiguous groups on the list of the tandem mass spectrometer using the processor; and
when at least one sentinel transition of the first group is detected by the tandem mass spectrometer, placing (360) a next group of the two or more contiguous groups identified by the at least one sentinel transition on the list and adjusting the at least one parameter of the separation device to a value identified by the at least one sentinel transition for the next group using the processor.

15. A computer program product (400) comprising a non-transitory and tangible computer-readable storage medium whose contents include a program with instructions configured to be executed on a processor (240) so as to cause a system (200) to perform the method (300) of claim 14, wherein the system comprises a separation device (210), an ion source (220), a tandem mass spectrometer (230), and the processor (240).

## Patentansprüche

1. System (200) zum Auslösen einer Gruppe von Mehrfachreaktionsüberwachungs-(MRM) -übergängen und Anpassen der Trennung für diese Gruppe, umfassend:
eine Trennungsvorrichtung (201), die eine oder mehrere bekannte Verbindungen aus einem Probengemisch trennt (310) und eine prozessorgesteuerte Anpassung mindestens eines Parameters der Trennungsvorrichtung während der Trennung erlaubt;
eine Ionenquelle (220), die die von der Trennungsvorrichtung empfangenen abgetrennten eine oder mehrere Verbindungen ionisiert (320), was einen Ionenstrahl aus einem oder mehreren Vorläufer-Ionen produziert;
ein Tandem-Massenspektrometer (230), das den Ionenstrahl von der Ionenquelle empfängt (330) und für jeden Zyklus einer Vielzahl von Zyklen an dem Ionenstrahl eine Reihe von MRM-Vorläufer-Ionen-zu-Produkt-Ionen-Übergängen ausführt, die aus einer Liste gelesen werden, wobei das Tandem-Massenspektrometer für jeden Übergang der Liste ein Vorläufer-Ion des Übergangs auswählt und fragmentiert und ein Produkt-Ion des Übergangs massenanalytisch analysiert; und
einen Prozessor (240) in Kommunikation mit dem Tandem-Massenspektrometer, der
zwei oder mehr zusammenhängende Gruppen von MRM-Übergängen zum Überwachen der einen oder mehreren bekannten Verbindungen empfängt (340), wobei jede Gruppe der zwei oder mehr zusammenhängenden Gruppen während der Vielzahl von Zyklen getrennt überwacht wird und mindestens einen Sentinel-Übergang einschließt, der eine nächste Gruppe der zwei oder mehr zusammenhängenden Gruppen identifiziert, die zu überwachen ist, und einen Wert für den mindestens einen Parameter für die nächste Gruppe identifiziert,
eine erste Gruppe der zwei oder mehr zusammenhängenden Gruppen auf der Liste des Tandem-Massenspektrometers platziert (350), und
wenn mindestens ein Sentinel-Übergang der ersten Gruppe durch das Tandem-Massenspektrometer detektiert wird, eine nächste Gruppe der zwei oder mehr zusammenhängenden Gruppen, die durch den mindestens einen Sentinel-Übergang identifiziert werden, auf der Liste platziert (360) und den mindestens einen Parameter der Trennungsvorrichtung an einen Wert anpasst, der durch den mindestens einen Sentinel-Übergang für die nächste Gruppe identifiziert ist.

2. System nach Anspruch 1, wobei die eine oder mehreren bekannten Verbindungen ein oder mehrere bekannte Peptide umfassen, die typischerweise von einem oder mehreren Proteinen verdaut werden.

3. System nach Anspruch 1, wobei die eine oder mehreren bekannten Verbindungen ein oder mehrere bekannte kleine Moleküle umfassen.

4. System nach Anspruch 1, wobei das eine oder die mehreren kleinen Moleküle Pestizide oder Missbrauchsdrogen umfassen.

5. System nach Anspruch 1, wobei die eine oder mehreren bekannten Verbindungen mindestens eine bekannte Verbindung des Gemischs und eine entsprechende isotopenmarkierte Version der mindestens einen bekannten Verbindung einschließen, die dem Gemisch in einer bekannten Konzentration zugesetzt wird, um als Standard für die Quantifizierung zu wirken.

6. System nach Anspruch 5, wobei die Trennungsvorrichtung eine Flüssigkeitschromatographie- (LC) -vorrichtung umfasst und der mindestens eine Parameter eine LC-Gradientenzeit der Trennung umfasst.

7. System nach Anspruch 6,
wobei die nächste Gruppe einen Übergang für die mindestens eine bekannte Verbindung der Mischung und einen Übergang für die isotopenmarkierte Version der mindestens einen bekannten Verbindung einschließt und
wobei der durch den mindestens einen Sentinel-Übergang identifizierte Wert für die LC-Gradientenzeit der Trennung auf einer vorbestimmten Wahrscheinlichkeit basiert, dass die Mischung eine Interferenz mit der mindestens einen bekannten Verbindung einschließt.

8. System nach Anspruch 7, wobei, falls die vorbestimmte Wahrscheinlichkeit hoch ist, der Wert die LC-Gradientenzeit erhöht.

9. System nach Anspruch 7, wobei, falls die vorbestimmte Wahrscheinlichkeit niedrig ist, der Wert die LC-Gradientenzeit verringert.

10. System nach Anspruch 7, wobei der Prozessor die LC-Gradientenzeit anpasst durch Anpassen eines Proportionalventils zwischen Lösungsmitteln.

11. System nach Anspruch 10, wobei die Lösungsmittel zwei Lösungsmittel umfassen.

12. System nach Anspruch **11,** wobei zwei Lösungsmittel ein wässriges Lösungsmittel und ein organisches Lösungsmittel umfassen.

13. System nach Anspruch 7,
wobei das Tandem-Massenspektrometer (230) weiter Verbindungsintensitäten für den Übergang für die mindestens eine bekannte Verbindung und Standardintensitäten für den Übergang für die isotopenmarkierte Version der mindestens einen bekannten Verbindung für einen oder mehrere Zyklen der Vielzahl von Zyklen detektiert, und
wobei das System weiter einen quantitativen Wert für die mindestens eine bekannte Verbindung aus den detektierten Verbindungsintensitäten, den detektierten Standardintensitäten und der bekannten Konzentration berechnet,
optional wobei die detektierten Standardintensitäten für den Übergang für die isotopenmarkierte Version der mindestens einen bekannten Verbindung verwendet werden, um eine Kalibrierungskurve zu erzeugen, und der quantitative Wert für die mindestens eine bekannte Verbindung unter Verwendung der Kalibrierungskurve berechnet wird.

14. Verfahren (300) zum Auslösen einer Gruppe von Mehrfachreaktionsüberwachungs- (MRM) -übergängen und Anpassen der Trennung für diese Gruppe, umfassend:
Trennen (310) einer oder mehrerer bekannter Verbindungen aus einem Probengemisch unter Verwendung einer Trennungsvorrichtung (210), die eine prozessorgesteuerte Anpassung mindestens eines Parameters der Trennungsvorrichtung während der Trennung erlaubt;
Ionisieren (320) der getrennten einen oder mehreren Verbindungen, die von der Trennungsvorrichtung empfangen werden, wobei ein Ionenstrahl aus einem oder mehreren Vorläufer-Ionen produziert wird;
Empfangen (330) des Ionenstrahls von der Ionenquelle (220) unter Verwendung eines Tandem-Massenspektrometers (230) und, für jeden Zyklus einer Vielzahl von Zyklen, Ausführen einer Reihe von MRM-Vorläufer-Ionen-zu-Produkt-Ionen-Übergängen an dem Ionenstrahl, die aus einer Liste unter Verwendung des Tandem-Massenspektrometers gelesen werden, wobei für jeden Übergang der Liste das Tandem-Massenspektrometer ein Vorläufer-Ion des Übergangs auswählt und fragmentiert und ein Produkt-Ion des Übergangs massenanalytisch analysiert;
Empfangen (340) von zwei oder mehr zusammenhängenden Gruppen von MRM-Übergängen zum Überwachen der einen oder mehreren bekannten Verbindungen unter Verwendung eines Prozessors (240), wobei jede Gruppe der zwei oder mehr zusammenhängenden Gruppen während der Vielzahl von Zyklen getrennt überwacht wird und mindestens einen Sentinel-Übergang einschließt, der eine nächste Gruppe der zwei oder mehr zusammenhängenden Gruppen identifiziert, die überwacht werden soll, und einen Wert für den mindestens einen Parameter für die nächste Gruppe identifiziert;
Platzieren (350) einer ersten Gruppe der zwei oder mehr zusammenhängenden Gruppen auf der Liste des Tandem-Massenspektrometers unter Verwendung des Prozessors; und
wenn mindestens ein Sentinel-Übergang der ersten Gruppe durch das Tandem-Massenspektrometer detektiert wird, Platzieren (360) einer nächsten Gruppe der zwei oder mehr zusammenhängenden Gruppen, die durch den mindestens einen Sentinel-Übergang identifiziert werden, auf der Liste und Anpassen des mindestens einen Parameters der Trennungsvorrichtung auf einen Wert, der durch den mindestens einen Sentinel-Übergang für die nächste Gruppe identifiziert wird, unter Verwendung des Prozessors.

15. Computerprogrammprodukt (400), umfassend ein nicht transitorisches und greifbares computerlesbares Speichermedium, dessen Inhalt ein Programm mit Anweisungen einschließt, die konfiguriert sind, um auf einem Prozessor (240) ausgeführt zu werden, um ein System (200) zu veranlassen, das Verfahren (300) nach Anspruch 14 durchzuführen, wobei das System eine Trennungsvorrichtung (210), eine Ionenquelle (220), ein Tandem-Massenspektrometer (230) und den Prozessor (240) umfasst.

## Revendications

1. Système (200) pour déclencher un groupe de transitions de surveillance de réactions multiples (MRM) et ajuster la séparation pour ce groupe, comprenant :
un dispositif (201) de séparation qui sépare (310) un ou plusieurs composés connus d'un mélange d'échantillon et permet un ajustement commandé par processeur d'au moins un paramètre du dispositif de séparation pendant la séparation ;
une source (220) d'ions qui ionise (320) les un ou plusieurs composés séparés reçus depuis le dispositif de séparation, produisant un faisceau d'ions d'un ou plusieurs ions précurseurs ;
un spectromètre (230) de masse en tandem qui reçoit (330) le faisceau d'ions provenant de la source d'ions et, pour chaque cycle d'une pluralité de cycles, exécute, sur le faisceau d'ions, une série de transitions MRM d'ions précurseurs vers ions produits lues à partir d'une liste, dans lequel pour chaque transition de la liste, le spectromètre de masse en tandem sélectionne et fragmente un ion précurseur de la transition et effectue une analyse de masse d'un ion produit de la transition ; et
un processeur (240) en communication avec le spectromètre de masse en tandem qui
reçoit (340) deux groupes contigus ou plus de transitions MRM pour surveiller les un ou plusieurs composés connus, dans lequel chaque groupe des deux groupes contigus ou plus est surveillé séparément pendant la pluralité de cycles et inclut au moins une transition sentinelle qui identifie un groupe suivant parmi les deux groupes contigus ou plus qui doit être surveillé et identifie une valeur pour l'au moins un paramètre pour le groupe suivant,
place (350) un premier groupe parmi les deux groupes contigus ou plus sur la liste du spectromètre de masse en tandem, et
lorsqu'au moins une transition sentinelle du premier groupe est détectée par le spectromètre de masse en tandem, place (360) un groupe suivant parmi les deux groupes contigus ou plus identifié par l'au moins une transition sentinelle sur la liste et ajuste l'au moins un paramètre du dispositif de séparation à une valeur identifiée par l'au moins une transition sentinelle pour le groupe suivant.

2. Système selon la revendication 1, dans lequel les un ou plusieurs composés connus comprennent un ou plusieurs peptides connus typiquement digérés à partir d'une ou plusieurs protéines.

3. Système selon la revendication 1, dans lequel les un ou plusieurs composés connus comprennent une ou plusieurs petites molécules connues.

4. Système selon la revendication 1, dans lequel les une ou plusieurs petites molécules comprennent des pesticides ou des drogues d'abus.

5. Système selon la revendication 1, dans lequel les un ou plusieurs composés connus incluent au moins un composé connu du mélange et une version à marquage isotopique correspondante de l'au moins un composé connu ajouté au mélange à une concentration connue pour agir comme étalon pour la quantification.

6. Système selon la revendication 5, dans lequel le dispositif de séparation comprend un dispositif de chromatographie liquide (LC) et l'au moins un paramètre comprend une durée de gradient LC de la séparation.

7. Système selon la revendication 6,
dans lequel le groupe suivant inclut une transition pour l'au moins un composé connu du mélange et une transition pour la version à marquage isotopique de l'au moins un composé connu et
dans lequel la valeur identifiée par l'au moins une transition sentinelle pour la durée de gradient LC de la séparation est basée sur une probabilité prédéterminée que le mélange inclue une interférence avec l'au moins un composé connu.

8. Système selon la revendication 7, dans lequel si la probabilité prédéterminée est élevée, la valeur augmente la durée de gradient LC.

9. Système selon la revendication 7, dans lequel si la probabilité prédéterminée est basse, la valeur diminue la durée de gradient LC.

10. Système selon la revendication 7, dans lequel le processeur ajuste la durée de gradient LC en ajustant une vanne proportionnelle entre les solvants.

11. Système selon la revendication 10, dans lequel les solvants comprennent deux solvants.

12. Système selon la revendication 11, dans lequel les deux solvants comprennent un solvant aqueux et un solvant organique.

13. Système selon la revendication 7,
dans lequel le spectromètre (230) de masse en tandem détecte en outre des intensités de composé pour la transition pour l'au moins un composé connu et des intensités étalons pour la transition pour la version à marquage isotopique de l'au moins un composé connu pour un ou plusieurs cycles de la pluralité de cycles, et
dans lequel le système calcule en outre une valeur quantitative pour l'au moins un composé connu à partir des intensités de composé détectées, des intensités étalons détectées et de la concentration connue,
facultativement dans lequel les intensités étalons détectées pour la transition pour la version à marquage isotopique de l'au moins un composé connu sont utilisées pour générer une courbe d'étalonnage et la valeur quantitative pour l'au moins un composé connu est calculée à l'aide de la courbe d'étalonnage.

14. Procédé (300) pour déclencher un groupe de transitions de surveillance de réactions multiples (MRM) et ajuster la séparation pour ce groupe, comprenant :
la séparation (310) d'un ou de plusieurs composés connus d'un mélange d'échantillon au moyen d'un dispositif (210) de séparation qui permet un ajustement commandé par processeur d'au moins un paramètre du dispositif de séparation pendant la séparation ;
l'ionisation (320) des un ou plusieurs composés séparés reçus depuis le dispositif de séparation, produisant un faisceau d'ions d'un ou plusieurs ions précurseurs ;
la réception (330) du faisceau d'ions provenant de la source (220) d'ions au moyen d'un spectromètre (230) de masse en tandem et, pour chaque cycle d'une pluralité de cycles, l'exécution, sur le faisceau d'ions, d'une série de transitions MRM d'ions précurseurs vers ions produits lues à partir d'une liste au moyen du spectromètre de masse en tandem, dans lequel, pour chaque transition de la liste, le spectromètre de masse en tandem sélectionne et fragmente un ion précurseur de la transition et effectue une analyse de masse d'un ion produit de la transition ;
la réception (340) de deux groupes contigus ou plus de transitions MRM pour surveiller les un ou plusieurs composés connus au moyen d'un processeur (240), dans lequel chaque groupe des deux groupes contigus ou plus est surveillé séparément pendant la pluralité de cycles et inclut au moins une transition sentinelle qui identifie un groupe suivant des deux groupes contigus ou plus qui doit être surveillé et identifie une valeur pour l'au moins un paramètre pour le groupe suivant,
le placement (350) d'un premier groupe des deux groupes contigus ou plus sur la liste du spectromètre de masse en tandem au moyen du processeur ; et
lorsqu'au moins une transition sentinelle du premier groupe est détectée par le spectromètre de masse en tandem, le placement (360) d'un groupe suivant des deux groupes contigus ou plus identifié par l'au moins une transition sentinelle sur la liste et l'ajustement de l'au moins un paramètre du dispositif de séparation à une valeur identifiée par l'au moins une transition sentinelle pour le groupe suivant au moyen du processeur.

15. Produit (400) de programme informatique comprenant un support de stockage non transitoire et tangible lisible par ordinateur dont le contenu inclut un programme avec des instructions configurées pour être exécutées sur un processeur (240) de manière à amener un système (200) à exécuter le procédé (300) selon la revendication 14, dans lequel le système comprend un dispositif (210) de séparation, une source (220) d'ions, un spectromètre (230) de masse en tandem et le processeur (240).
